# EUROPEAN PATENT SPECIFICATION

(11) **EP 3 960 174 B1**
(45) Date of publication and mention of the grant of the patent: **30.10.2024**
(21) Application number: 20193376.9
(22) Date of filing: 28.08.2020
(51) Int. Cl.: A61K 31/445, A61K 31/40, A61P 1/14, A61P 9/04, A61P 9/06, A61P 21/00, A61P 25/28, C07D 207/12

(54) **PARASYMPATHETIC ACTIVE PHENYL-GLYCOSID-SUBSTITUTED HYDROXY-METHYL-PIPERIDINE AND -PYRROLIDINE ALKALOIDS FOR MEDICINAL USE**
PARASYMPATHISCHE AKTIVE PHENYL-GLYCOSID-SUBSTITUIERTE HYDROXY-METHYL-PIPERIDIN- UND -PYRROLIDIN-ALKALOIDE ZUR MEDIZINISCHEN VERWENDUNG
ALCALOÏDES DE PYRROLIDINE OU DE HYDROXY-MÉTHYL-PIPÉRIDINE SUBSTITUÉ PAR PHÉNYL-GLYCOSIDE AVEC UNE ACTIVITÉ PARASYMPATHÉTIQUE POUR UTILISATION MÉDICALE

(43) Date of publication of application: 02.03.2022
(73) Proprietor: Westfälische Wilhelms-Universität Münster, 48149 Münster (DE)
(72) Inventor: HENSEL, Andreas, 48161 Münster (DE); DÜFER, Martina, 48161 Münster (DE); SYMMA, Nico, 48161 Münster (DE); HAKE, Alexander, 48147 Münster (DE)
(74) Representative: Michalski Hüttermann & Partner Patentanwälte mbB

(56) References cited:
- CN-A- 106 349 141
- S NISHINO ET AL: "Muscle atonia is triggered by cholinergic stimulation of the basal forebrain: implication for the pathophysiology of canine narcolepsy", THE JOURNAL OF NEUROSCIENCE, vol. 15, no. 7, 1 July 1995 (1995-07-01), US, pages 4806 - 4814, XP055771415, ISSN: 0270-6474, DOI: 10.1523/JNEUROSCI.15-07-04806.1995
- CHESHIRE WILLIAM P JR ET AL: "Drug-induced hyperhidrosis and hypohidrosis - Incidence, prevention and management", DRUG SAFETY, ADIS PRESS, AUCKLAND, NZ, vol. 31, no. 2, 1 January 2008 (2008-01-01), pages 109 - 126, XP009189658, ISSN: 0114-5916
- POPE C ET AL: "Pharmacology and toxicology of cholinesterase inhibitors: uses and misuses of a common mechanism of action", ENVIRONMENTAL TOXICOLOGY AND PHARMACOLOGY, ELSEVIER, AMSTERDAM, NL, vol. 19, no. 3, 1 May 2005 (2005-05-01), pages 433 - 446, XP027640516, ISSN: 1382-6689, [retrieved on 20050501]

## Description

The present invention relates to a compound according to the following formula 1: 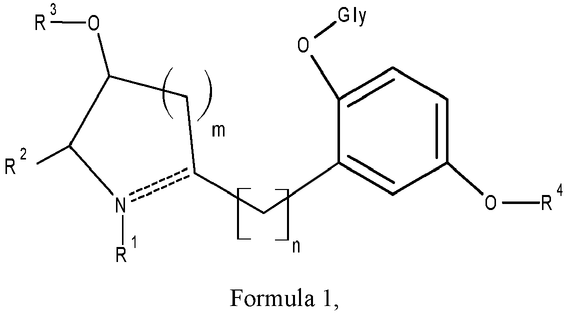 or a pharmaceutically acceptable salt thereof for use as a medicament, wherein n = 7 - 12; m = 1 or 2; R¹ = H, C1-C3 alkyl or void, and in case R¹ is void the nitrogen is integrated in the N-heterocycle via a double bond and in case R¹ is H or C1-C3 alkyl the nitrogen is integrated in the N-heterocycle via a single bond; R² = C1-C3 alkyl; R³ = H or -CO-R⁵ and R⁵ is C1-C3 alkyl; R⁴ = H or -CO-R⁶ and R⁶ is C1-C3 alkyl; Gly = mono- or disaccharide. In addition, the present invention relates to a compound and a pharmaceutical composition comprising said compound for use in the treatment of Alzheimer's disease, atonic dysregulation of smooth muscles cells and smooth muscles, tachycardia and diminished contractility of the heart, to increase reduction of anticholineric side effects induced by drugs within pharmacotherapy.

Acetylcholine (ACh) is an essential transmitter and modulator for inter cell-cell communication and interaction in humans and many animals. As a function of the included cell types and cell location different tasks are regulated by the presence or absence of ACh. For muscle activation the substance is released by nerve cells and interacts with muscle cells by special ACh receptors. In the autonomic nervous system the substance acts simultaneously as an internal transmitter and as a product released by the parasympathetic nervous system. In addition, ACh is the primary neurotransmitter of the parasympathetic nervous system. Also the brain comprises a number of cholinergic areas, wherein the presence of ACh influences emotional and cognitive functions like stimulation, concentration, memory and motivation. Central ACh stimulus can result in activation or inhibition of neurons.

In order to establish the required functional lifetime of the released transmitter, ACh finds it counterpart in an ACh degrading enzyme, i.e. acetylcholinesterase (AChE). Therefore, ACh is released from the presynaptic neuron into the synaptic cleft, binds to corresponding ACh receptors on the post-synaptic membrane and is afterwards enzymatically degraded into choline and acetate. The "right" dynamic equilibrium between ACh generation, release and degradation form the basis of a normal and healthy cellular and body function. Consequently, based on the broad and important influence on the body many drug substances have been developed affecting or influencing cholinergic transmission.

Some approaches directed to influence the ACh equilibrium in-vivo can also be found in the patent literature.

EP 2 999 480 B1, for instance, discloses a composition of extracts from *Hippeastrum papilio* comprising galanthamine, narwedine and haemanthamine obtainable by conventional procedures for liquid-liquid extraction of alkaloids, and characterized in that it contains the following components as determined by integration of their GC-MS peak areas and expressed as a percentage of their total ion current: galanthamine from 40% to 98%, haemanthamine from 0.01% to 35%, narwedine from 0.01% to 10%, and other alkaloids of homolycorine-, and/or tazettine-, and/or monthanine- and/or lycorine-type from 0.001% to 35%, and non-alkaloid plant metabolites from 0.001 to 1 %, and in that it is derived from the total extracts from dry herbage of *Hippeastrum papilio.*

Furthermore, EP2892563B1 discloses a N-(2-(6-fluoro-1H-indol-3-yl)ethyl)-3-(2,2,3,3-tetra-fluoropropoxy)benzylamine or a pharmaceutically acceptable salt thereof for use in treating Alzheimer's disease by improving or augmenting the effect of an acetylcholinesterase inhibitor comprising administering an effective daily dose of said compound to a patient in need of such treatment, wherein the effective daily dose administered to the patient is between 30 and 60 mg.

In addition, WO 2013 168 090 A1 discloses the use of effective amount of any one of the *Pelargonium graveolens* constituents selected from a group consisting of: (S)(-)citronellol, linalool, menthone and isomenthone or any combination thereof, in the preparation of a medicament for treating a mammal suffering from or susceptible to a neurodegenerative condition which can be improved or prevented by inhibition of acetylcholinesterase (AChE).

Further information with respect to cholinesterase inhibitors and disease management can for instance be found in CN 106 349 141 A, S Nishino et al.: "Muscle atonia is triggered by cholinergic stimulation of the basal forebrain: implication for the pathophysiology of canine narcolepsy", The Journal of Neuroscience, vol. 15, no. 7, 1 July 1995 (1995-07-01), pages 4806-4814, XP055771415, US ISSN: 0270-6474, DOI: 10.1523/JNEUROSCl.15-07-04806.1995; Cheshire William P JR et al.: "Drug-induced hyperhidrosis and hypohidrosis - Incidence, prevention and management", Drug Safety, Adis Press, Auckland, NZ, vol. 31, no. 2, 1 January 2008 (2008-01-01), pages 109-126, XP009189658, ISSN:0114-5916 and Pope C et al.: "Pharmacology and toxicology of cholinesterase inhibitors: uses and misuses of a common mechanism of action", Environmental Toxicology And Pharmacology, Elsevier, Amsterdam, NL, vol. 19, no. 3, 1 May 2005 (2005-05-01), pages 433-446, XP027640516, ISSN: 1382-6689 [retrieved on 2005-05-01].

Nevertheless, besides the already existing approaches to influence ACh concentrations under in vivo conditions in humans or animals, there is still the need to provide further compounds, which are able to positively influence cholinergic transmission.

Therefore, it is the task of the current invention to overcome, at least in part, the drawbacks of the state of the art. Especially, it is a task of the current invention to provide a compound group useful as drug/medicament for use in humans or veterinary medicine. Furthermore, it is a task of the current invention to provide a compound group useful as compounds for use in the treatment of diseases related to a reduced ACh concentration or a reduced ACh response as further specified in claim 12 and pharmaceutical compositions comprising said compounds.

The above mentioned task is solved by compounds for use as medicaments according to the independent claim 1. In addition, the task is further solved by compounds for use in specific treatments according to the features of the second medical use claim and a pharmaceutical composition comprising said inventive compounds. Preferred embodiments of the invention are also defined by the features of the dependent claims, by features disclosed in the description and in the figures, wherein a feature aggregation of separated parts is within the scope of the invention, unless explicitly excluded.

It is within the scope of the present invention to disclose a compound according to the following formula 1: 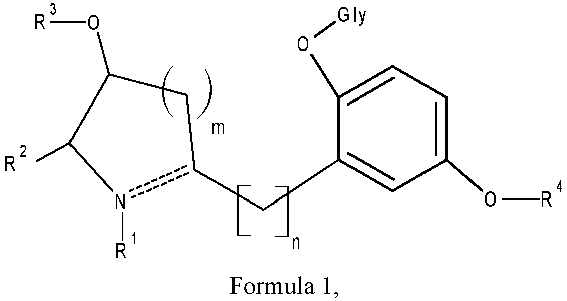 or a pharmaceutically acceptable salt thereof, wherein n = 7 - 12; m = 1 or 2; R¹ = H, C1-C3 alkyl or void, and in case R¹ is void the nitrogen is integrated in the N-heterocycle via a double bond and in case R¹ is H or C1-C3 alkyl the nitrogen is integrated in the N-heterocycle via a single bond; R² = C1-C3 alkyl; R³ = H or -CO-R⁵ and R⁵ is C1-C3 alkyl; R⁴ = H or -CO-R⁶ and R⁶ is C1-C3 alkyl; Gly = mono- or disaccharide; for use as a medicament.

Surprisingly, it has been found that the specific alkaloids according to formula 1 can be used as human or veterinary medicament. Pharmacological tests based on the compounds revealed that the compounds are able to stimulate the parasympathetic nervous system by modulation of the ACh receptors. This has been shown by using isolated organs of mice within ex vivo experiments. In addition, it could be shown that the compounds comprise a significant and concentration dependent AChE inhibition. Therefore, the compounds are able to positively influence conditions related to diseases related to reduced ACh concentration levels or reduced ACh response. Furthermore, based on a toxicological in vitro assessment, including different cell lines (human liver cells, human epithelial cells keratinocytes, epithelial intestinal cells), it has been shown that the compounds do not influence the cellular proliferation over a wide concentration range (1 to 100 µM) and therefore can be regarded as non-toxic. Compared to the existing drug alternatives in that field the compounds are not only direct para-sympathomimetic drugs by AChE inhibition, but can also be considered as positive or negative modulators of the cell membrane associated ACh receptor. This means, that the compounds actively change the ACh induced quantitative response, mediated by the receptor. In addition, the ex vivo and in vitro data reveal, that besides the influence on the receptor also AChE activity is inhibited, resulting in a two-sided cholinergic effect. Based on these findings the compounds can be used in the treatment of a variety of different human or animal diseases as well as in pest control. The latter use is reasonable, because many insects, protozoa or parasitic helminths comprise an ACh regulated neuronal system.

The inventive group of compounds is suitable for and intended to be used as a medicament. The alkaloids according to formula 1 are effective in treating human and animal subjects, also including non-mammalian animals like helminths, protozoa and insects. Therefore, in the case of humans and mammalian animals the medicament is able to prevent or ease an illness based on ACh dysregulation. For insects or helminths the medicament can be used for pest control.

The compounds according to formula 1 can be used as such or in the form of a pharmaceutically acceptable salt. It is obvious to the skilled artisan that based on the protonable/de-protonable moieties in the Markush formula the compound may be neutral or charged, depending on the respective pH. Therefore, as a function of the chemical surrounding and environment the compounds may comprise a positive or negative charge, wherein the counter-ions may, for instance, be halogen, alkali, alkaline earth or organic acid counter-ions.

The compounds according to formula 1 comprise two cyclic structures, connected by an alkyl chain. The alkyl chain may comprise from 7 up to 12 -CH₂- groups in-between the ring structures.

The non-aromatic N-heterocycle may be a 5 or a 6 membered cycle as expressed by the index m. In case m equals 1 a five membered N-heterocyle and in case m equals 2 a 6 membered N-heterocycle is formed. The basic structure of the 5 membered N-heterocycle is known as a pyrrolidine and the 6 membered N-heterocycle is known as a piperidine.

For the substituent R¹ three different alternatives are feasible. On the one hand R¹ can represent an atom or a group of atoms, i.e. H or C1-C3 alkyl. On the other hand, R¹ can be void or absent. Based on the presence or absence of this group also the ring structure is changing. In case wherein R¹ is void or absent the nitrogen is integrated in the N-heterocycle via a double bond. If a group is present at the N-atom in the heterocycle a single bond is necessary for charge compensation in the N-heterocycle, only. Therefore, for this moiety four alternatives are possible:

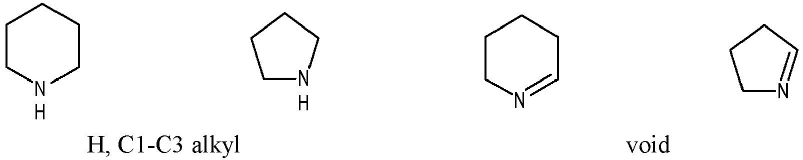

The further substituents are R² = C1-C3 alkyl; R³ = H or -CO-R⁵ and R⁵ is C1-C3 alkyl; R⁴ = H or -CO-R⁶ and R⁶ is C1-C3 alkyl; Gly = mono- or disaccharide, wherein substituent R² is directly bound to the N-heterocycle and can be a lower alkyl like methyl, ethyl, propyl. The substituents R³ and R⁴ are bound to the cycles via an -O- linkage and may form either a hydroxyl or an ester-group. On the aromatic ring structure a mono- or disaccharide is attached, wherein suitable mono- or di-saccharides are known to the skilled artisan. It is assumed that the glycosidic group can also be eliminated and removed by intestinal or systemic catabolic transformation in the organism not changing the activity of the molecule. It is further assumed that the deglycosylated compounds are able to penetrate biological barriers (e.g. blood brain barrier, intestinal epithelia etc.) in the organisms better than the glycosylated compounds.

In a preferred embodiment of the compound for use n is equal to 9 or 10. Without being bound to the theory it is assumed that the overall length of the alkyl chain spacer in between the hetero and the aromatic ring influences the magnitude of interaction of the compound and the ACh receptor as well as the interaction of the compound and AChE. For compounds comprising alkyl chains of 9 or 10 CH₂-groups the receptor and enzyme interaction seems to be favorable, resulting in more distinct physiological effects. This can result in different degrees of interaction with the cell membrane bound ACh receptor, leading to variable modulation of the receptor activity. Additionally, the alkyl chain length will have relevant influence on the physicochemical properties of the compounds for use (e.g. polarity, solubility, distribution coefficient etc.) which again will influence pharmacokinetic as well as pharmacodynamic properties

In a further preferred embodiment of the compound for use Gly can be selected from the group consisting of glucose, mannose, galactose, xylose, rhamnose, arabinose, fucose, glucuronic acid, rutinose. Based on the overall solubility of the compound and the biocompatibility above mentioned group of sugars has been found advantageous. The higher degree of polarity of the glycosylated compounds bears advantages for optimized galenical formulations (e.g. aqueous solutions, solution for injection etc.).

Within a further preferred aspect of the compound for use Gly can be glucose. The biocompatibility and solubility contribution of a monosaccharide in the form of glucose has been found favorable. It is further assumed that the glucose moiety can be removed after oral intake of the compound for use by glucosidases in the intestinal tract, leading to a deglycosylated compound with higher degree of absorption over the intestinal barrier. It is assumed that the glucose moiety of the compound for use can be recognized also by specific glucose transporters in in various tissues and cells of the organism, leading to a higher degree of absorption by interaction with the glucose transporter, which transports the glucosylated compounds over and through the biological barrier.

In a preferred aspect of the compound for use R² can be methyl. Especially a very short chain alkyl substitution at the N-heterocycle is able to positively influence the interaction with the receptor and the AChE interaction of the compound.

Within a further preferred characteristic of the compound for use R³ can be -CO-CH₃. In order to achieve the "right" solubility of the compound into the cell surrounding and for better transport of the compounds over barriers (e.g. epithelia, cell membranes, blood brain barrier etc.) it can be helpful to introduce an ester group at the N-heterocycle. This may result in an inactivation of the compound, but increases the bioavailability after oral administration and also the penetration over the blood brain barrier into the central nervous system. The compound is more lipophilic, and, therefore, penetrates better lipophilic barriers. The transformation to the physiologically active hydroxyl-form can be achieved by esterases, present in the cell surrounding or in case of an oral delivery form is used in the intestine or liver, wherein the pro-drug is converted to the physiologically active entity.

In a preferred embodiment of the compound for use R⁴ can be H. The resulting hydroxyl group at the aromatic system can enhance the interaction of the compound with the AChE or the ACh receptor via hydrogen bond.

Within a preferred aspect of the compound for use R¹ can be H and m can be 2. A favorable interaction of the compound, with the ACh receptor and AChE can be achieved in cases, wherein the N-heterocycle comprises a piperidine structure. The better physiologic interactions may result from the specific steric or electronic structure at this position.

In another embodiment of the compound for use according R¹ can be void and m is 1. A favorable interaction of the compound, with the ACh receptor and AChE can be achieved in cases, wherein the N-heterocycle comprises a partial pyrrolidine structure and a double bond in the ring. The better physiologic interactions may result from the specific steric or electronic structure at this position.

Within a preferred aspect of the compound for use R⁴ can be -CO-CH₃. In order to achieve the "right" solubility for the transport of the compound into the cell surrounding and for better transport of the compounds over barriers (e.g. epithelia, cell membranes, blood brain barrier etc.), it can be helpful to introduce an ester group at the aromatic ring. This may result in an inactivation of the compound, but increases the bioavailability after oral administration and also the penetration over the blood brain barrier into the central nervous system. The compound is more lipophilic, and, therefore, penetrates better lipophilic barriers. The transformation to the physiologically active phenolic-form can be achieved by esterases, present in the cell surrounding or in case of an oral delivery is used in the intestine or liver, wherein the pro-drug is converted to the physiologically active specie.

In a preferred embodiment of the compound for use the compound can be selected from the group consisting of 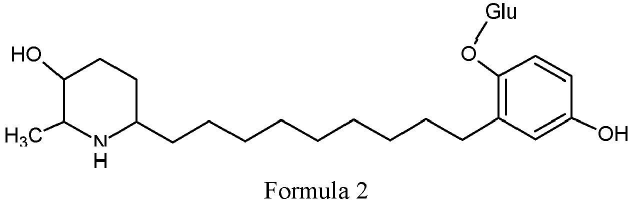 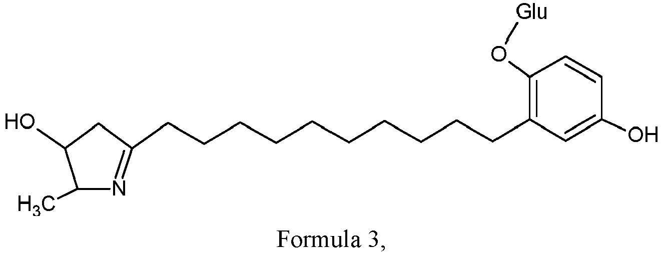 wherein Glu is glucose, or mixtures or salts thereof. The two above depicted structures have been found very effective in ex vivo and in vitro testing. Significant influences on ACh response are achieved at low compound concentrations, indicating a high affinity to AChE and inhibition of the enzyme activity and a favorable interaction with the ACh receptor. The compounds are stable in a physiological environment and non-toxic.

Furthermore, it is within the scope of the current invention to disclose the use of the inventive compounds in the treatment of diseases related to reduced ACh concentration levels or reduced ACh response as defined in claim 12.

Because of the physiological effects of the compounds it has been found helpful to use the compounds in treatments of diseases, wherein ACh concentrations are lower than required or usual. The ACh depletion may origin from an increased AChE concentration or activity, or from reduced ACh synthesis or from an alteration of the receptor response. In these cases, an administration of an effective amount of the compounds is able to reduce the physiological effects of the malfunction or disbalance The compounds may further be helpful in cases, wherein the disbalance is induced by chemical compounds.

In addition, the inventive compounds can be used in the treatment of diseases related to reduced ACh concentration levels or reduced ACh response, wherein the diseases are selected from the group consisting of Alzheimer's disease, atonic dysregulation of smooth muscles cells and smooth muscles, tachycardia and diminished contractility of the heart, to increase reduction of anticholineric side effects induced by drugs within pharmacotherapy. Many diseases affecting the central nervous system (CNS) are related to a reduced ACh concentration or a reduced ACh receptor response. Inter alia Alzheimer disease and some other forms of dementia, also schizophrenia and psychoses can be named in that group. In cases were cognitive function or mental stress is induced by a dysbalance of central neurotransmitters (e.g. hyperactivity of adrenergic, noradrenergic, dopaminerc or serontonergic system, leading to a relative underactivity of the cholinergic system) increase in ACh concentration could reduce symptoms. In addition, further diseases as e.g. atonic bladder dysfunction, tachycardic arrhythmia, reduced cardiac contractility, hypertension, ophthalmologic disorders associated with reduced ACh titers, atonic disorders of smooth muscle cells, smooth muscles and the gall bladder, improvements in saliva production and secretion, gastric juice secretion, bronchial secretion, perspiration are, at least in part, dependent on the respective ACh response. Sweeting is mainly controlled by ACh, therefore stimulation of the ACh system by the inventive compounds can lead to hyperhidrosis, which again is favorable during e.g. common cold infections and can also be used for forced detoxification and elimination of compounds over the skin. Therefore, it is possible to positively influence dysfunctional states by administering the inventive group of compounds. Additionally, the inventive group of compounds can also be used to treat unwanted anticholinergic side effects of drugs within pharmacotherapy. E.g. many antidepressants reduce the activity of the cholinergic system as an unwanted side effect, which is known to be in general a big burden for the patients; typical symptoms are dry mouth or cardiac problems or reduced sweeting or reduced body temperature etc. These conditions can be treated by application of the compounds described in this invention.

Besides targeting human or mammalian animals it is further possible to use the inventive group of substances as inhibitor for the vitality of insects in general. It is possible to use the compounds for the elimination of insects in human or veterinary related areas. Furthermore, it is possible to use the inventive group of substances as helminth or protozoa inhibitor.

Additionally, a pharmaceutical composition comprising the inventive compounds for use and a pharmaceutically acceptable excipient is within the scope of the current invention. The compounds according to the invention can be integrated in a pharmaceutical composition by adding one or more suitable excipients. Excipients can either be liquid or solid, depending on the chosen drug delivery. It is possible to dissolve the compounds in a pharmaceutically acceptable solvent and to formulate an i.v. or i.m delivery form, or a different formulation for any parenteral application. Furthermore, it is possible to formulate an oral delivery by further adding tableting substances. Based on the chemical and physical properties several different delivery forms can be generated. With respect to the further advantages of the pharmaceutical composition it is especially referenced to the advantages of the inventive medical use. ceutical composition it is especially referenced to the advantages of the pharmaceutical composition and the inventive medical use.

### Examples

### I. Synthesis of the compounds

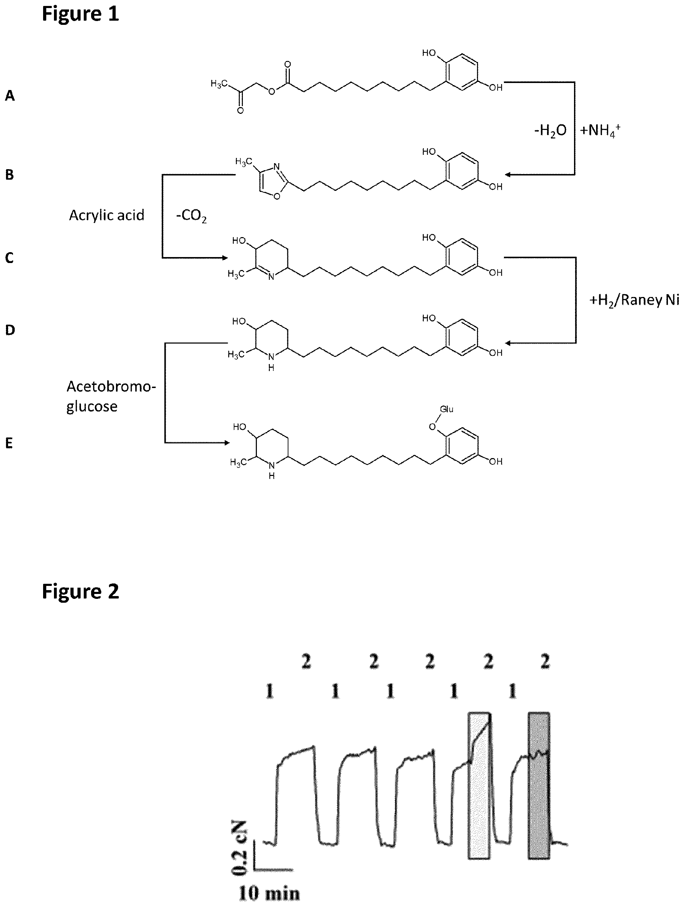

The claimed compounds can be synthesized according to a route depicted in figure 1.

A cyclization reaction is performed on compound A in the presence if NH₄⁺, followed by elimination of water, resulting in the formation of the N,O heterocyle comprising compound B. In the presence of acrylic acid the 5 membered N,O-heterocycle is transformed to an unsaturated, 6 membered N-heterocyclce (compound C). The heterocycle is hydrogenated in the presence of Raney Ni and Hydrogen to yield compound D, followed by a glycolysation step assisted by acetobromoglucose to yield compound E.

### II. Extraction from Plant

The inventive compounds can be isolated and purified from the herbal material and especially from the flowers of species from the genus *Tilia* (plant family Malvaceae).

### IIa. Reagents

If not stated otherwise, solvents and reagents were of analytical quality and obtained from VWR International (Darmstadt, Germany). Consumables were obtained from Sarstedt (Nümbrecht, Germany). Water was produced by a Millipore simplicity 185 system (Merck, Darmstadt, Germany).

### IIb. MS Measurements

UHPLC-ESI-qTOF-MSMS analysis was performed using a Dionex Ultimate 3000 RS Liquid Chromatography System on a Dionex Acclaim RSLC 120, C18 column (2.1 × 100 mm, 2.2 µm) column at a temperature of 40°C. Injection volume: 10 µL. Flow rate: 0.4 mL/min. Samples were dissolved in methanol/water (9:1 v/v) to a concentration of 10 mg/mL. The binary gradient comprised of (A) water with 0.1% formic acid and (B) acetonitrile with 0.1% formic acid and was designed as follows: 0.48 - 9.00 min linear from 15 to 38 % B; 9.00-9.02 min linear from 38 to 100 % B; 9.02-15.00 min isocratic at 100 % B; 15.00-15.10 min linear from 100 to 10 % B; 15.10-20.00 min isocratic at 10 % B. Eluted compounds were detected using a Dionex Ultimate DAD-3000 RS over a wavelength range of λ = 200-400 nm and a Bruker Daltonics micrOTOF-QII time-of-flight mass spectrometer equipped with an Apollo electrospray ionization source in positive mode at 3 Hz over a mass range of m/z 50-1500 using the following instrument settings: nebulizer gas nitrogen: 3.5 bar; dry gas nitrogen: 9 L/min, 200 °C; capillary voltage: 4500 V; end plate offset: -500 V; transfer time: 100 µs, prepulse storage: 6 µs, collision energy: 8 eV. MS/MS experiments were performed for verification of ion identity via fragmentation pattern. MS/MS scans were triggered by AutoMS2 settings within a range of m/z 50-1500, using a collision energy of 30-70 eV and collision cell RF of 130 Vpp.

### IIc. NMR Measurements

One- and two-dimensional NMR spectroscopic experiments were carried out using an Agilent DD2 spectrometer (Agilent Technologies, Santa Clara, USA) at 600 MHz (¹H) or 151 MHz (¹³C) at 299 K. Samples were dissolved in methanol-d4 (≥ 99.8 atom % D) from Sigma-Aldrich (Steinheim, Germany). Chemical shifts were referenced to the residual signals of non-deuterated solvent (¹H: 4.870 ppm and 3.31 ppm; ¹³C: 49.00 ppm).

### IId. Extraction Procedure

Dried and cut lime flowers (1.3 kg) were extracted in aliquots of 100 g two times with each 1 L of a mixture of cold acetone:water (7:3 v/v) under exclusion of direct light. After removal of acetone by evaporation, chlorophyll was removed from the extracts by liquid-liquid partitioning of the remaining aqueous phase with petroleum ether (5 × 600 mL for each extraction step), resulting in 225 g (17 % w/w, related to the starting material) of crude extract A. Collected and dried *Tilia cordata* flowers (68 g) and *Tilia platyphyllos* flowers (151 g) were extracted according to the same protocol yielding 12 g (17% w/w) aqueous extract TP and 30 g (20% w/w) aqueous extract TC.

Subsequently, 80 g of crude extract A were fractionated using a Bond Elute Plexa PCX cationic exchange solid phase extraction (SPE) (200 mg, 6 mL) from Agilent Technologies (Folsom, USA). The crude extract was dissolved in 2% H₃PO₄ in methanol/water (1:1 v/v) at a concentration of 100 mg/mL. The SPE separation was performed in accordance with the specification served by the manufacturer. After equilibration with methanol 100% and water 100%, 3 mL of the sample solution was loaded on the cartridge and subsequently eluted with each 6 mL of 2% formic acid in water, methanol/acetonitrile (1:1 v/v), 5% NH₃ in methanol/acetonitrile (1:1 v/v) and 5% NH₃ in methanol/water (1:1 v/v). Every elution step lead to a fraction, which was each monitored by UHPLC-ESI-MS on RP-18 stationary phase, as described below. *Tilia* alkaloids eluted with 5% NH₃ in methanol/acetonitrile (1:1 v/v), yielding 1.42 g (1.77 % (w/w) related to the crude extract) of basic fraction B.

A Sephadex LH-20 column (345 × 30 mm; bed volume: 244 mL) was prepared for fractionation of SPE fraction B. 1.42 g of SPE fraction B was dissolved 15 mL 5 % NH₃ in ethanol/water (1:1 v/v), and successively separated in steps of 5 mL. Isocratic elution with 250 mL 5 % NH3 in ethanol/water (1:1 v/v) at a flow of 0.8 mL/min yields 25 fractions á 10 mL. Monitoring these fractions via UHPLC-ESI-MS as described below showed that *Tilia* alkaloids together with other substances eluted after 180 to 210 mL. Fractions were combined and yielded 367 mg (0.46 % (w/w) related to the crude extract) of basic fraction C.

Preparative HPLC was performed to isolate the single compounds from basic fraction C using Waters Quaternary Gradient Module 2545, Photodiode Array Detector 2998, Autosampler 2707, Waters Prep Degasser and Waters Fraction Collector III. Software: Waters Chrom-Scope v1.40 Beta (Waters, Milford, MA, USA). Nucleodur C18 HTec (5 µm, 250 × 21 mm) served as stationary phase. Mobile phase gradient: (A) water/trifluoroacetic acid (98:2 v/v) and (B) acetonitrile/trifluoroacetic acid (98:2 v/v): 0 to 16 min: linear from 0 to 18 % B; 16 to 56 min: linear from 18 to 30 % B; 56 to 64 min: linear from 30 to 100 % B; 64 to 68 min: isocratic at 100 % B; 68 to 72 min: linear from 100 to 0 % B; 72 to 80 min: isocratic at 0 % B; column temperature: room temperature; Flow: 10.0 mL/min; injection volume: 1000 µL.

SPE, Sephadex LH-20 and preparative HPLC-fractions were monitored for the presence of *Tilia* alkaloids using UHPLC-ESI-MS system and a stationary phase ACQUITY UPLC HSS T3 (1.8 µm, 2.1 × 100 mm) (Waters, Milford, U.S.A.). Mobile phase gradient: A: water with 0.1 % formic acid; B: acetonitrile with 0.1 % formic acid), t_{R} 0-4 min: linear from 0 to 18 % B; 4-14 min: linear from 18 to 30 % B; 14-16 min: linear from 30 to 100 % B; 16-17 min: isocratic at 100 % B; 17-18 min: linear from 100 to 0 % B; 18-20 min: isocratic at 0 % B; column temperature: 40 °C; flow rate: 0.5 mL/min; injection volume: 4 µL. Eluted compounds were detected using a Waters PDA eλ over a wavelength range of 200-800 nm and a Waters QDa mass spectrometer over a range of in the positive ion mode with a cone voltage of 15 V and a capillary voltage of 0.8 V.

### IIe. Identification

Three different compounds can be isolated:

### IIe. 1 Compound 1 - tiliine A

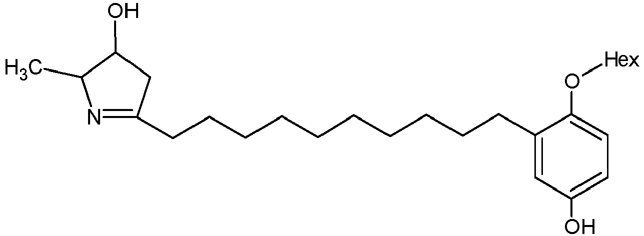

3.86 mg of compound **1** had been isolated as yellow oil from 367 mg of fraction C. HR-MS analysis revealed *m*/*z* 510.3070 [M+H]⁺, correlating to the molecular formula of C₂₇H₄₄NO₈⁺. MS² analysis at 30 to 70 eV produced a fragment with *m*/*z* 348.2491 (C₂₁H₃₄NO₃⁺) and a neutral loss of 162 u (C₆H₁₀O₅), indicating the presence of a hexose moiety. This was also proven by carbohydrate-typical resonances in the respective NMR spectra.

¹³C NMR signals at δ_{C} 153.45, 150.33, 135.13, 118.46, 117.35, 113.84 ppm indicated the presence of an aromatic system, for which two positions are linked to an oxygen (δ_{C} 153.45, 150.33 ppm) and one position substituted by an additional carbon (δ_{C} 135.13 ppm). *Para-*substitution was deduced from ¹H NMR signals at δ_{H} 7.01 ppm, (d, *J* = 8.7 Hz, 1H), δ_{H} 6.57 ppm (d, *J* = 3.0 Hz, 1H) and δ_{H} 6.54 ppm (dd, *J* = 8.6, 3.0 Hz, 1H) and confirmed by the typical roof effect of the signals δ_{H} 6.54 ppm to δ_{H} 7.01 ppm.

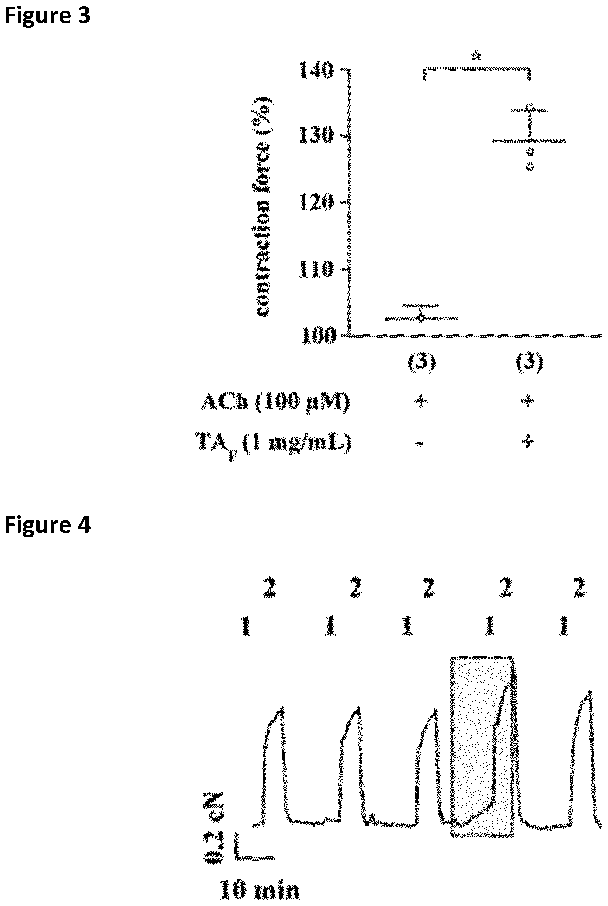

It was interesting to detect also a carbon with a strong downfield shift (δ_{C} 195.08 ppm), which was supposed to be connected to a nitrogen via double bond linkage. Based on HMBC data (Figure 3) a coupling of this strongly downfield shifted carbon with the protons of a CH₂ group at δ_{H} 1.92 and 2.02 ppm linked to a carbon at δ_{C} 25.39 ppm and to two CH groups at δ_{H} 4.04 and 3.86 ppm linked to carbons at δ_{C} 64.06 and 57.25 ppm are detectable. Together with the respective COSY data the structure of a 3,4-dihydro-2H-pyrrole is proposed. Additionally, substitution of the heterocycle by a methyl group was found, based on COSY correlations from δ_{H} 3.86 ppm proton to protons of a methyl group at δ_{H} 1.42 ppm connected to a carbon at δ_{C} 15.29 ppm. Also substitution at C3 of the heterocycle was observed, based on typical downfield shift of C3 δ_{C} 64.04 ppm. This was assumed to be due to hydroxylation of C3. Furthermore, HMBC data reveal the linkage of the δ_{C} 195.08 ppm carbon to an alkyl chain of 10 carbons (starting at carbon δ_{C} 38.80 ppm, ending at carbon δ_{C} 30.98 ppm). This alkyl chain, attached on one side to the heterocycle, is also connected to the phenolic part of the molecule, which is proven by HMBC coupling of the aromatic carbons with the protons from carbon at δ_{C} 30.98 ppm.

Further analysis of MS² fragmentation pattern confirmed the proposed structural features and the different building blocks of the molecule (hexose, dihydroxy-methyl-phenol, alkyl chain and hydroxyl-methyl-dihydro-pyrrole) could be verified by the typical MS fragmentation sequence

After elimination of a hexose moiety, a neutral loss of 124 u was observed, indicative for a methyl-hydroquinone structure, as proposed by NMR analysis. The resulting fragment (*m*/*z* 224.2011, C₁₄H₂₆NO⁺) represented a methyl-hydroxyl-di-substituted dihydropyrrole moiety with a 9-carbon alkyl chain. Subsequently, a multiple elimination of methylene groups starting from *m*/*z* 224.2011 is apparent (mass difference of Δu = 14), until only the heterocycle at *m*/*z* 112.0756 (C₆H₁₀NO⁺) is left. This confirms the proposed 2-methyl-3-hydroxy-3,4-dihydro-2H-pyrrole moiety. Additionally, an elimination of the hydroxyl group in position 3 (Δu = 18) and subsequent formation of a double bond is obvious at any stage of the fragmentation pathway.

The molecule is supposed to consist of four main structural elements: a 2-methyl-3-hydroxy-3,4-dihydro-2H-pyrrole heterocycle, a C10 alkyl chain, a hydroquinone and a hexose.

Pale yellow oil. HR-ESI-MS: *m*/*z* 510.3070 [M+H]⁺ (calculated molecular formula: [C₂₇H₄₄NO₈]⁺). ¹H NMR (600 MHz, methanol-d4): δ 7.01 (d, J = 8.7 Hz, 1H), δ 6.57 (d, J = 3.0 Hz, 1H), 6.54 (dd, J = 8.6, 3.0 Hz, 1H), 4.72 (d, J = 7.8 Hz, 1H), 4.04 (dt, J = 4.8, 2.4 Hz, 1H), 3.88 (dd, J = 12.0, 2.1 Hz, 1H), 3.86 (m, 1H), 3.70 (dd, J = 12.0, 5.1 Hz, 1H), 3.43 (m, 2H), 3.37 (m, 2H), 2.68 (m, 1H), 2.67 (m, 2H), 2.58 (dt, J = 13.5, 7.6 Hz, 1H), 2.02 (dt, J = 14.0, 4.2 Hz, 1H), 1.92 (m, 1H), 1.70 (m, 2H), 1.59 (t, J = 7.3 Hz, 2H), 1.42 (d, J = 7.0 Hz, 3H), 1.35 - 1.29 (m, 12H). ¹³C NMR (151 MHz, methanol-d4): δ 195.08, 153.45, 150.33, 135.13, 118.46, 117.35, 113.84, 103.95, 78.38, 78.02, 75.20, 71.54, 64.06, 62.64, 57.24, 38.80, 31.22, 30.98, 30.41, 30.36, 30.32, 30.09, 30.06, 30.01, 27.55, 25.39, 15.29.

### IIe.2 Compound 2 - tiliamine A

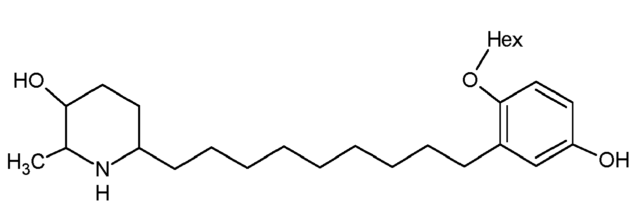

5.33 mg of compound **2** were isolated from a single HPLC peak. HR-MS revealed *m*/*z* 512.3234, corresponding to the calculated molecular formula of C₂₇H₄₆NO₈⁺. Again in MS² spectra a neutral loss of 162 was observed, indicating the presence of a hexose moiety. Detailed analysis of 1D and 2D NMR revealed similarities in the structural features of compound **1** with regard to a *β*-hexose, which was determined by CZE as glucose. The glucose moiety is again linked to a 1,4-hydroquinone, substituted at C2" by an alkyl chain. Differences are obvious in the NMR signals of the heterocycle moiety. In contrast to compound 1 no carbons with a strong downfield shift are detectable. Instead, an additional ¹³C signal at δ_{C} 58.73 ppm with one proton at δ_{H} 3.06 ppm was detected, indicating a CH group, in direct neighborhood to the nitrogen. HMBC and COSY signals revealed a connection to the alkyl chain on the one hand, and on the other hand to a heterocycle of five carbons and one nitrogen. From this the heterocycle is assessed to be a C2/C3-substituted piperidine. The positions C2 (δ_{C} 57.53 ppm) and C3 (δ_{C} 65.82 ppm) are substituted to a methyl group and a hydroxyl group, respectively. For clarification of relative stereochemistry at position C2 and C3 of the heterocycle H,H-NOESY experiments were performed, which clearly indicated cis-configuration of the H2/H3 protons. From these investigations the structure of the piperidine alkaloid **2** (tiliamine A) can be deduced.

Pale yellow oil. HR-ESI-MS: *m*/*z* 512.3234 [M+H]⁺ (calculated molecular formula: [C₂₇H₄₆NO₈]⁺). ¹H NMR (600 MHz, methanol-d4): δ 7.01 (d, J = 8.7 Hz, 1H), 6.57 (d, J = 3.0 Hz, 1H), 6.55 (dd, J = 8.7, 3.0 Hz, 1H), 4.73 (d, J = 7.7 Hz, 1H), 3.88 (dd, J = 12.0, 2.1 Hz, 1H), 3.84 (dt, J = 3.4, 1.6 Hz, 1H), 3.70 (dd, J = 12.0, 5.2 Hz, 1H), 3.43 (m, 2H), 3.37 (m, 2H), 3.23 (m, 1H), 3.06 (tdd, J = 11.7, 8.5, 4.2 Hz, 1H), 2.69 (m, 1H), 2.59 (dt, J = 13.4, 7.7 Hz, 1H), 1.96 (m, 1H), 1.82 (m, 1H), 1.72 (m, 2H), 1.67 (m, 1H), 1.59 (m, 2H), 1.57 (m, 1H), 1.44 (ddd, J = 10.3, 5.8, 2.6 Hz, 1H), 1.37 - 1.26 (m, 14H). ¹³C NMR (151 MHz, methanol-d4): δ 153.45, 150.32, 135.15, 118.46, 117.34, 113.84, 103.95, 78.38, 78.02, 75.20, 71.54, 65.82, 62.64, 58.73, 57.53, 34.74, 31.26, 31.00, 30.99, 30.54, 30.53, 30.44, 30.42, 30.40, 26.28, 23.58, 15.89.

### IIe.3 Compound 3 - acetyl-tiliamine A

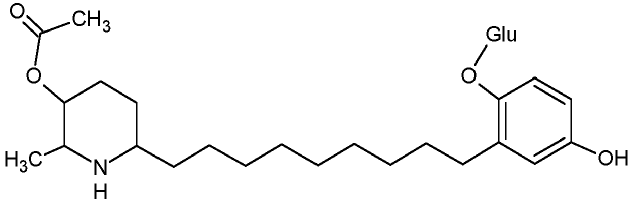

Compound **3** (2.61 mg) eluted after compound **2** on RP18 preparative HPLC, which indicates a more lipophilic character. LC-HRMS analysis revealed *m*/*z* 554.3325 [M+H]⁺ which corresponds to molecular formula: C₂₉H₄₆NO₉⁺. A mass difference of 42 u compared to compounds **2** could represent an additional acetyl group. Analysis of 1D and 2D NMR data reveals again manifold similarities to the above described compounds, regarding the existence of a hexose, a 2-alkyl hydroquinone moiety and an alkyl chain. Also, similar signals of a 2-methyl-2-hydroxy-piperidine ring are detectable, but in contrast to compound **2** a downfield shift of the proton and the carbon of position C3 is obvious, indicating an acetyl group at the respective C3 hydroxyl group. Furthermore, in ¹³C spectrum additional signals at δ_{C} 171.31 and 20.69 ppm confirm the hypothesis of an acetyl group. As in the HMBC spectrum a coupling of H-3 with the carbonyl-carbon δ_{C} 171.31 can be observed, an acetylation of C3 hydroxyl group is proven.

Pale yellow oil. HR-ESI-MS: *m*/*z* 554.3325 [M+H]⁺ (calculated molecular formula: [C₂₉H₄₆NO₉]⁺). ¹H NMR (600 MHz, methanol-d4) δ 7.01 (d, J = 8.7 Hz, 1H), 6.57 (d, J = 3.0 Hz, 1H), 6.55 (dd, J = 8.7, 3.0 Hz, 1H), 5.10 (q, J = 2.6 Hz, 1H), 4.74 (d, J = 7.7 Hz, 1H), 3.89 (dd, J = 12.0, 2.2 Hz, 1H), 3.71 (dd, J = 11.9, 5.2 Hz, 1H), 3.50 (qd, J = 6.7, 2.0 Hz, 1H), 3.44 (m, 2H), 3.38 (m, 2H), 3.18 (m, 1H), 2.69 (dt, J = 13.4, 7.6 Hz, 1H), 2.59 (dt, J = 13.5, 7.7 Hz, 1H), 2.15 (s, 3H), 2.06 (dq, J = 14.0, 3.0 Hz, 1H), 1.94 (ddt, J = 10.2, 6.8, 3.2 Hz, 1H), 1.82 (dddd, J = 14.7, 13.5, 4.3, 2.6 Hz, 1H), 1.72 (m, 1H), 1.62 (m, 1H), 1.60 (m, 2H), 1.57 (m, 1H), 1.48 (m, 1H), 1.40 (m, 1H), 1.36 (m, 10H), 1.29 (d, J = 6.7 Hz, 3H). ¹³C NMR (151 MHz, methanol-d4) δ 171.25, 153.46, 150.32, 135.15, 118.47, 117.34, 113.84, 103.95, 78.38, 78.02, 75.21, 71.54, 69.16, 62.64, 58.48, 55.76, 34.62, 31.25, 30.99, 30.51, 30.50, 30.42, 30.39, 30.36, 28.03, 26.23, 24.08, 20.69, 15.52.

Summarizing the results of the respective preparation and isolation methods, three unknown alkaloids could be identified from Lime flower. Tiliine A, tiliamine A and acetyl-tiliamine A. Samples from *Tilia platyphyllos* and *Tilia cordata* flowers, which were extracted and analyzed separately by HPLC-ESI-qTOF, showed the existence of all recently identified *Tilia* alkaloids in flowers of both species.

### III In-vivo, In-vitro Measurements

### IIIa Mouse Trachea

For assessment of the pharmacologic effects of the inventive compounds on the cholinergic system of the smooth musculature the mouse trachea model was chosen. A mixture of compounds 1 and 2 (as defined in the experimental section) was dissolved in water at a concentration of 1 mg/ml and the solution was added to the organ bath (Mayflower, Hugo Sachs Elektronik, March, D). Figure 2 depicts the results of 5 independent measurements, starting from the left. In the first three tests a contraction of the trachea was induced by adding 100 µM ACh. A contraction of the trachea is visible on the given force scale. Between each experiment a rinsing step is performed as indicated by the arrows. The fourth measurement is performed after addition of the compound mixture and equilibration of the bath for 10 minutes. It can be seen, that the trachea response is larger compared to the first three cycles. The last experiment reveals, that after a rinsing step approximately the same response is generated by ACh compared to the first three control measurements. The latter is an indicator that the pharmacological effect of the test compounds is reversible.

The quantitative differences in the induced contraction force are depicted in figure 3. It becomes visible, that the trachea contraction force is approximately 25% higher (p ≤ 0.05) after supplementing the inventive compound.

Figure 4 displays another trachea experiment. The overall setup is similar to the above described experiment. The first three peaks are control peaks originated from contractions based on ACh alone. In the fourth cycle the inventive compound was added at the ACh induced contraction peak and remained in the bath for 5 minutes. It is visible that the contraction force is significantly increased after compound addition. The fifth cycle exhibits, that after washing the "standard" ACh induced contraction force is re-established, indicating a reversable influence of the inventive compounds on smooth muscle contraction.

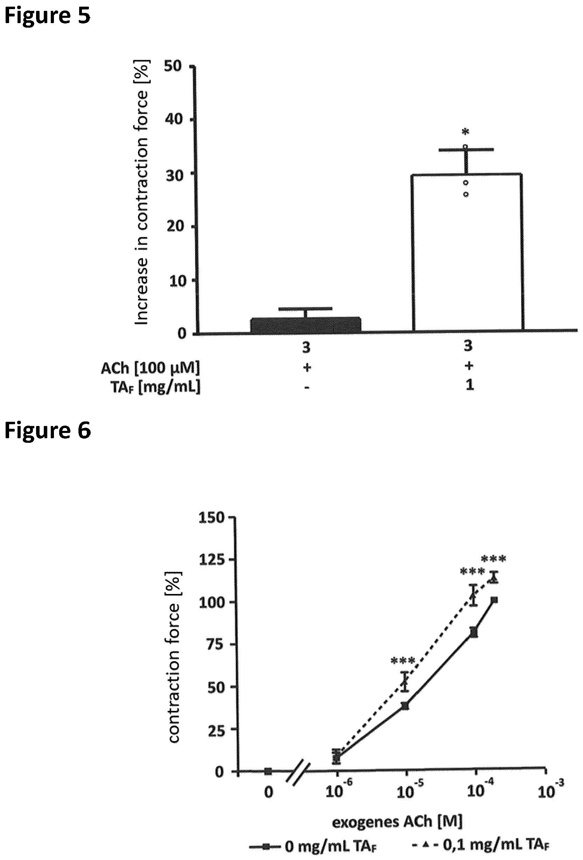

The quantitative physiological effect of the inventive compounds on the achievable maximum of the ACh contraction is displayed in figure 5. It can be seen that the contraction force is significantly increased by approximately 25%.

Figure 6 shows the ACh dose dependent effects on trachea contraction force of a control (solid line) and a bath also comprising 1 mg/ml of a mixture of inventive compound 1 and 2. The ACh concentrations are given on a logarithmic scale. Each result is given as an average also comprising the standard deviation. The data reveal that there is a significant difference between standard and experiment starting from 10⁻⁶ molar ACh.

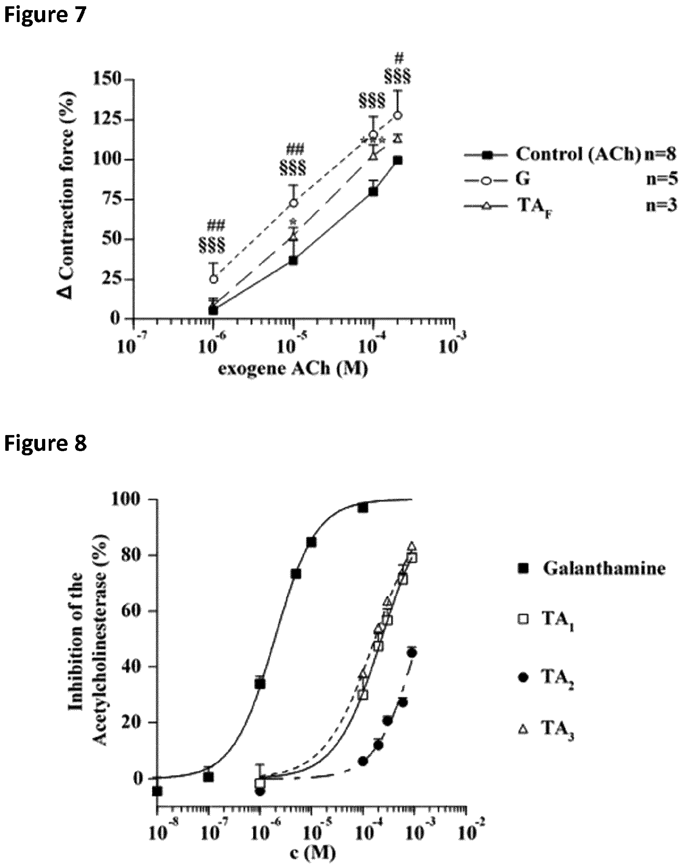

Figure 7 displays the pharmacological effects of galantamine, a "gold" standard in ACh response modulation, on the force response of a mouse trachea. Galantamine was added to the bath 10 minutes prior to ACh addition. The experiment is comparable to the experiment depicted in and described for figure 2. A comparison of figure 7 and figure 6 reveals, that galantamine comprises a response already at lower ACh levels and the overall increase in contraction force is somewhat higher.

The mouse trachea model data reveal, that in an in-vivo model the inventive compounds are able to alter the ACh response of smooth muscle cells. This becomes visible by an increased contraction force after supplementing the inventive compounds to the bath.

### IIIb AChE inhibition

For assessing the ability of the inventive compounds 1, 2 and 3 (as defined in the experimental section) to also interact with AChE, the single substances were tested with an acetylcholinesterase Inhibitor Screening Kit (Sigma Aldrich, Taufkirchen, D). The test assay is based on the Ellman method, wherein Acetylthiocholin after hydrolysis reacts with 5,5'-Dithio-bis-2-nitrobenzoic acid forming the colored 5-thio-2-nitrobenzoate. The latter can be detected in an UV-experiment at 412 nm.

The experiments were performed in a 96 well plate. An AChE concentration of 90 u/L was employed. Galantamine was used as a positive control at 0.01, 0.1, 1, 5, 10 und 100 µM. The inventive compounds were tested in concentrations of 1, 100, 200, 300, 600 und 900 µM. A well without inhibitor was used as a negative control. For assessing the UV-absorption after 10 minutes reaction time a plate reader (Clariostar BMG LABTECH GmbH, Ortenberg, D) was used.

Figure 8 shows the inhibition profile of three compounds according to the invention and the "gold" standard galantamine as a function of the inhibitor concentration. It can be deduced that the gold standard comprises a higher potential to inhibit AChE (IC₅₀ 2 µM), based on the earlier onset at lower concentrations and the overall better inhibitory effect. Nevertheless, the inventive compounds do also show a significant concentration dependent inhibition comprising IC₅₀ values of 231 µM (compound 1) and 172 µM (compound 2), respectively. For the third compound, compound 3, the 50% inhibition level was not achieved in this experiment, presumably based on the acetyl-moiety substitution. Nevertheless, based on the increased lipophilicity it can be assumed that the in-vivo bioavailability of this compound after oral delivery will be higher and the acetyl group will be cleaved from the molecule either in the intestine or the liver. After transformation the biologically active form is generated from the pro-drug and the same inhibition effects are expected.

### IIIc Tox screening

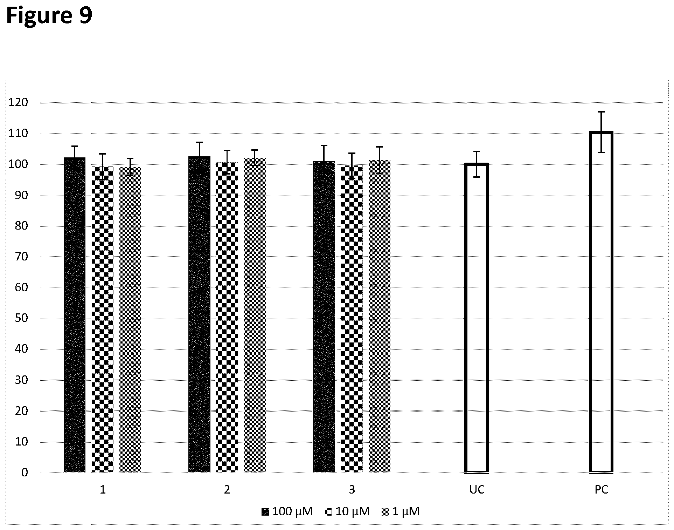

In order to evaluate the toxicity profile (influence on cellular proliferation by BrdU incorporation assay) of the inventive compounds, different compound concentrations (1, 10 and 100 µM) were tested. Control UC was defined as the untreated control = 100% reference value; a further stimulation control was treated with 10% fetal calf serum, which typically increases the cellular proliferation, and this control group PC proved validity of the test system also for proliferation-inducing effects on different human cell lines (HepG2 liver cells, HaCaT keratinocytes and CaCo-2 intestinal cells). Figure 9 shows exemplarily, that up to 100 µM no statistical significant reduction in cell proliferation can be detected for HaCaT cells. Similar results are obtained for the other cell lines. This result indicates that the inventive compounds are very biocompatible.

## Claims

1. Compound according to the following formula 1: 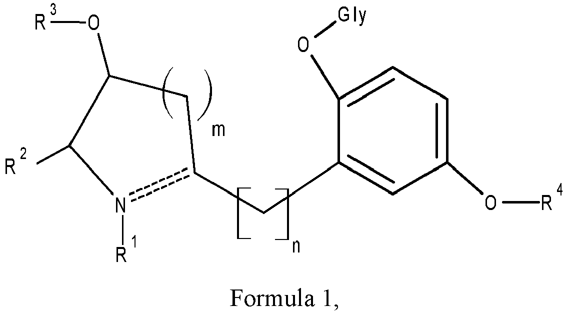
or a pharmaceutically acceptable salt thereof, wherein
n=7- 12;
m = 1 or 2;
R¹ = H, C1-C3 alkyl or void, and in case R¹ is void the nitrogen is integrated in the N-heterocycle via a double bond and in case R¹ is H or C1-C3 alkyl the nitrogen is integrated in the N-heterocycle via a single bond;
R² = C1-C3 alkyl;
R³ = H or -CO-R⁵ and R⁵ is C1-C3 alkyl;
R⁴ = H or -CO-R⁶ and R⁶ is C1-C3 alkyl;
Gly = mono- or disaccharide;
for use as a medicament.

2. Compound for use according to claim 1, wherein n = 9 or 10.

3. Compound for use according to any one of the preceding claims, wherein Gly is selected from the group consisting of glucose, mannose, galactose, xylose, rhamnose, arabinose, fucose, glucuronic acid, rutinose.

4. Compound for use according to any one of the preceding claims, wherein Gly is glucose.

5. Compound for use according to any one of the preceding claims, wherein R² is methyl.

6. Compound for use according to any one of the preceding claims, wherein R³ is -CO-CH₃.

7. Compound for use according to any one of the preceding claims, wherein R⁴ is H.

8. Compound for use according to any one of the preceding claims, wherein R¹ is H and m = 2.

9. Compound for use according to any one of claims 1-7, wherein R¹ is void and m = 1.

10. Compound for use according to any one of the preceding claims, wherein R⁴ is -CO-CH₃.

11. Compound for use according to any one of the preceding claims, wherein the compound is selected from the group consisting of 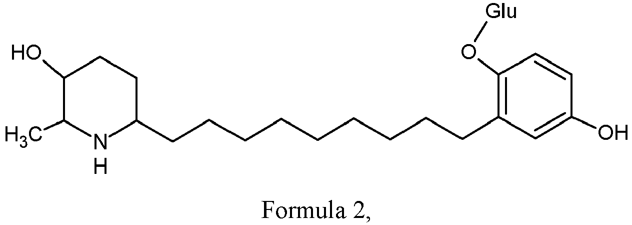 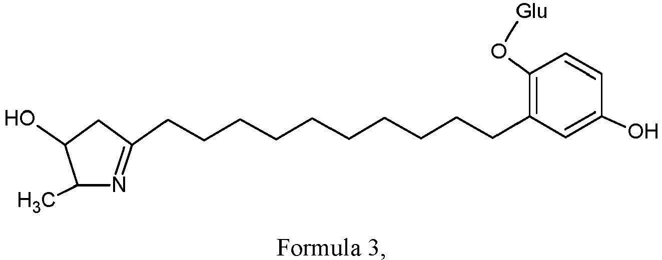 wherein Glu is glucose, or mixtures or salts thereof.

12. Compound for use according to any one of the preceding claims for use in the treatment of diseases related to reduced acetylcholine concentration levels or reduced acetylcholine response, wherein the disease is selected from the group consisting of Alzheimer's disease, atonic dysregulation of smooth muscles cells and smooth muscles, tachycardia and diminished contractility of the heart, to increase reduction of anticholineric side effects induced by drugs within pharmacotherapy.

13. Pharmaceutical composition comprising a compound for use according to any one of the claims 1-11 and a pharmaceutically acceptable excipient.

## Patentansprüche

1. Verbindung nach der folgenden Formel 1: 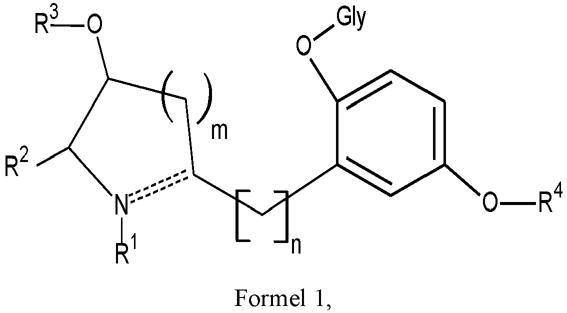
oder ein pharmazeutisch akzeptables Salz davon, wobei
n = 7 - 12;
m = 1 or 2;
R¹ = H, C1-C3 Alkyl oder Nichts, und im Fall, dass R¹ Nichts ist, ist der Stickstoff im N-Heterozyklus über eine Doppelbindung integriert und im Falle das R¹ H oder ein C1-C3 Alkyl ist, ist der Stickstoff über eine Einzelbindung im N-Heterozyklus integriert;
R² = C1-C3 Alkyl;
R³ = H or -CO-R⁵ and R⁵ is C1-C3 Alkyl;
R⁴ = H or -CO-R⁶ and R⁶ is C1-C3 Alkyl;
Gly = Mono- oder Disaccharid;
zur Verwendung als Medikament.

2. Verbindung zur Verwendung nach Anspruch 1, wobei n = 9 oder 10 ist.

3. Verbindung zur Verwendung nach einem der vorhergehenden Ansprüche, wobei Gly ausgesucht ist aus der Gruppe bestehend aus Glucose, Mannose, Galactose, Xylose, Rhamnose, Arabinose, Fucose, Glucuronsäure, Rutinose.

4. Verbindung zur Verwendung nach einem der vorhergehenden Ansprüche, wobei Gly Glucose ist.

5. Verbindung zur Verwendung nach einem der vorhergehenden Ansprüche, wobei R² Methyl ist.

6. Verbindung zur Verwendung nach einem der vorhergehenden Ansprüche, wobei R³ -CO-CH₃ ist.

7. Verbindung zur Verwendung nach einem der vorhergehenden Ansprüche, wobei R⁴ H ist.

8. Verbindung zur Verwendung nach einem der vorhergehenden Ansprüche, wobei R¹ H und m = 2 ist.

9. Verbindung zur Verwendung nach einem der Ansprüche 1 - 7, wobei R¹ Nichts ist und m = 1.

10. Verbindung zur Verwendung nach einem der vorhergehenden Ansprüche, wobei R⁴ -CO-CH₃ ist.

11. Verbindung zur Verwendung nach einem der vorhergehenden Ansprüche, wobei die Verbindung ausgesucht ist aus der Gruppe bestehend aus 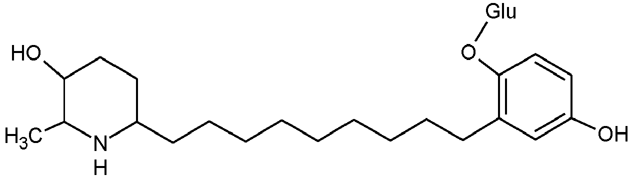 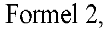 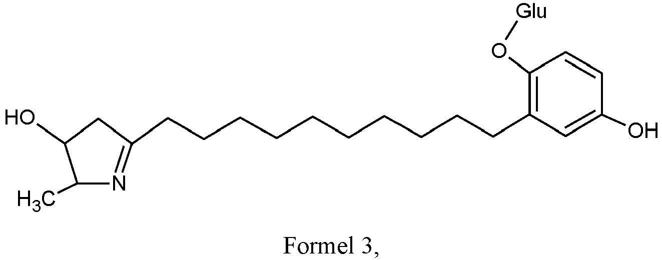 wobei Glu Glucose ist, oder Mischungen oder Salze davon.

12. Verbindung zur Verwendung nach einem der vorhergehenden Ansprüche zur Verwendung in der Behandlung von Krankheiten verbunden mit reduzierten acetylcholinen Konzentrationsniveaus oder einer reduzierten acetylcholinen Antwort, wobei die Krankheiten ausgesucht sind aus der Gruppe bestehend aus Alzheimer, atonische Dysregulierung der glatten Muskelzellen oder der glatten Muskeln, Tachykardie und verminderte Kontraktilität des Herzens, zur Verbesserung der Reduzierung der anticholinen Nebenwirkungen induziert durch Medikamente während der Pharmakotherapie.

13. Pharmazeutische Zusammensetzung umfassend eine Verbindung zur Verwendung nach einem der Ansprüche 1-11 und ein pharmazeutisch verträglicher Hilfsstoff.

## Revendications

1. Composé selon la formule 1 suivante : 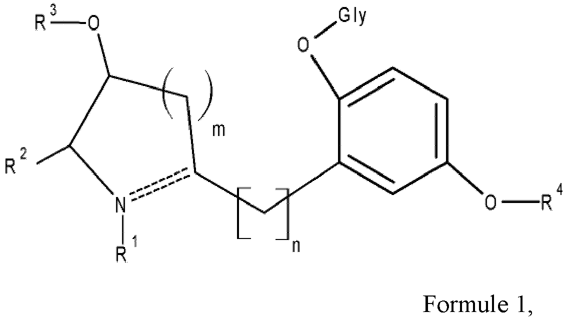
ou un sel pharmaceutiquement acceptable de celui-ci, dans lequel
n = 7 à 12 ;
m = 1 ou 2 ;
R¹ = H, un alkyle en C1-C3 ou rien, et dans le cas où R¹ ne représente rien, l'azote est intégré au N-hétérocycle au moyen d'une double liaison, et dans le cas où R¹ représente H ou un alkyle en C1-C3 l'azote est intégré au N-hétérocycle au moyen d'une liaison simple ;
R² = un alkyle en C1-C3 ;
R³ = H ou -CO-R⁵ et R⁵ représente un alkyle en C1-C3 ;
R⁴ = H ou -CO-R⁶ et R⁶ représente un alkyle en C1-C3 ;
Gly = un mono- ou disaccharide ;
pour une utilisation comme médicament.

2. Composé pour une utilisation selon la revendication 1, dans lequel n = 9 ou 10.

3. Composé pour une utilisation selon l'une quelconque des revendications précédentes, dans lequel Gly est choisi dans le groupe constitué par le glucose, le mannose, le galactose, le xylose, le rhamnose, l'arabinose, le fucose, l'acide glucuronique, le rutinose.

4. Composé pour une utilisation selon l'une quelconque des revendications précédentes, dans lequel Gly représente le glucose.

5. Composé pour une utilisation selon l'une quelconque des revendications précédentes, dans lequel R² représente un méthyle.

6. Composé pour une utilisation selon l'une quelconque des revendications précédentes, dans lequel R³ représente -CO-CH₃.

7. Composé pour une utilisation selon l'une quelconque des revendications précédentes, dans lequel R⁴ représente H.

8. Composé pour une utilisation selon l'une quelconque des revendications précédentes, dans lequel R¹ représente H et m = 2.

9. Composé pour une utilisation selon l'une quelconque des revendications 1 à 7, dans lequel R¹ ne représente rien et m = 1.

10. Composé pour une utilisation selon l'une quelconque des revendications précédentes, dans lequel R⁴ représente -CO-CH₃.

11. Composé pour une utilisation selon l'une quelconque des revendications précédentes, dans lequel le composé est choisi dans le groupe constitué par 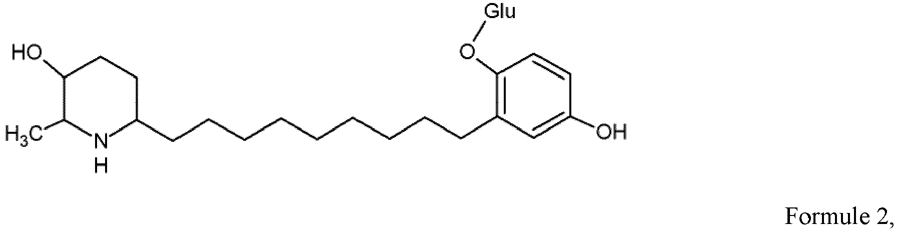 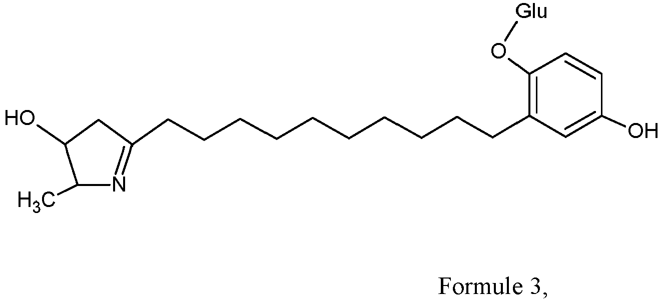 dans lequel Glu représente le glucose, ou des mélanges ou des sels de celui-ci.

12. Composé pour une utilisation selon l'une quelconque des revendications précédentes pour une utilisation dans le traitement de maladies liées à des niveaux de concentration réduits en acétylcholine ou une réponse en acétylcholine réduite, dans lequel la maladie est choisie dans le groupe constitué par la maladie d'Alzheimer, le dérèglement atonique des cellules musculaires lisses et des muscles lisses, la tachycardie et la contractilité réduite du coeur, pour augmenter la réduction des effets secondaires anticholinergiques induits par des médicaments inclus dans une pharmacothérapie.

13. Composition pharmaceutique comprenant un composé pour une utilisation selon l'une quelconque des revendications 1 à 11 et un excipient pharmaceutiquement acceptable.
